# EUROPEAN PATENT APPLICATION

(11) **EP 4 169 445 A1**
(43) Date of publication of application: **26.04.2023**
(21) Application number: 21203818.6
(22) Date of filing: 20.10.2021
(51) Int. Cl.: A61B 5/107, A61B 90/00

(54) **DEVICE FOR MEASURING DEPTH OF AN INTERNAL FEATURE**

(71) Applicant: Lys Medical SA, 6041 Gosselies (BE)
(72) Inventor: PATERNOTTE, Jean, 1300 Limal (BE); WITMEUR, Marius, 1180 Brussels (BE); MERTENS, Benjamin, 1332 Rixensart (BE); BLANC, Loic, 1180 Uccle (BE); GEUENS, Nicolas, 1410 Waterloo (BE)
(74) Representative: AWA Benelux

(57) **Abstract**

A device (100) for measuring advancement of an instrument (210) inside a body, the device being configured to be detachably attached to a guide (200) for the instrument, the guide comprising an inlet port (220) through which the instrument (210) can access the body. The device comprises a base (110) comprising attachment means (111.1, 111.2, 111.3) for attachment to the guide (200) so that the base (110) remains fixed with respect to the inlet port (220) when attached, and a sliding member (120) comprising a coupling means (130) configured to releasably engage with the instrument (210) so that the sliding member moves integrally with the instrument when coupled. The device comprises a translational joint (112.1, 112.2) allowing the sliding member to translate relative to the base. One of the base and the sliding member comprise a ruler section (121) with a graduated scale configured to co-operate with the other one of the base and the sliding member to perform a measurement of a distance of movement.

## Description

### Technical Field

The present invention relates to a device for measuring the longitudinal size of an anatomical feature inside a patient and to a medical manipulator, in particular an endoscope fitted with said device.

### Background Art

Measuring a stenosis based on the displacement of an endoscopic element such as a catheter tip has been performed in the medical field for a while.

Traditionally such measuring is performed by two persons. An assistant measures the displacement of the catheter and a physician handles the endoscope.

In order to streamline the operation, in particular the reduction of operators, US 5239982 discloses the determination of the length of a stenosis using a catheter tip. The physician can read on a transparent gauge formed on a angioscope (specific case of endoscope) the location of a reference mark formed on the catheter tube that is inserted in the angioscope. The displacement of the catheter tip can be followed by comparing the position of the reference mark to a graduate scale formed on the transparent gauge. The length of the stenosis is related to the distance along which the mark has moved along the graduated scale after the catheter tip has been moved through stenotic lesion.

One drawback of this solution is that the catheter must comprise one or more marks. Another is that the transparent element distorts the view of the mark(s) thereby reducing the precision of the measurement. Furthermore, the transparent body cannot be integrated in a modular way into the angioscope. Finally, the transparent element cannot be easily cleaned.

An electronic depth measurement device is known from US 2007/0250006. The measuring device includes a housing is attached to the entrance of the working channel of an endoscope. The housing accommodates a guide adapted to receive an elongated instrument, such as a catheter, and a sensor module. The guide guides the elongated instrument along the sensor module and further into the working channel of the endoscope. The sensor module includes an optical image sensor or an optical rotary encoder to sense movement of the elongated instrument within the guide. The housing further accommodates a display for displaying the measurement and a reset button to reset the displayed measurement.

Although such a device provides ease of measurement, it has heretofore not found general application because it is difficult to clean and make sterile again, while being too costly for disposal after single use. Moreover, the measuring device must remain attached to the endoscope handle while the instrument is inserted in the working channel, and therefore, the device hinders handling of the instrument when no measurement is performed.

### Aims of the Invention

The invention aims to provide a solution to at least one drawback of the teaching provided by the prior art.

More specifically, the invention aims to provide a measurement device which is simple to use and is more ergonomic (e.g. does not hinder operation when no measurement is performed).

### Summary of the Invention

According to an aspect of the present disclosure, there is therefore provided a device as set out in the appended claims. A device according to aspects of the present disclosure is configured to measure a distance of travel of an elongate instrument travelling inside a body, and thereby measuring a longitudinal size of a feature inside the body. The device is adapted to be possibly detachably attached to an instrument guide. The guide comprises an inlet port receiving the elongate instrument and providing access to the body, and can comprise an internal lumen communicating with the inlet port. The guide can be a manipulator, such as an endoscope, particularly a handle of the endoscope, and the internal lumen can be a working channel of the endoscope. Alternatively, the guide can be an access point for inserting instruments inside a body, such as a trocar, or any other rigid or flexible instrument guide. The instrument can be a catheter, a guide wire or any other suitable elongate instrument capable of being received in the internal lumen.

The device comprises a base and a sliding member. The base comprises attachment means or an attachment system for possibly detachable attachment to the guide, so that the base remains fixed with respect to the inlet port when attached. The device comprises a translational joint, in particular a linear joint, allowing the sliding member to translate relative to the base. The sliding member comprises a coupling means or a coupling part configured to engage with the instrument. The coupling part advantageously allows the sliding member to move integrally with the instrument when the coupling part is engaged with the instrument. The coupling part is advantageously configured as a releasable engagement, allowing the sliding member to be disengaged from the instrument, e.g. when the coupling part is released.

One of the base and the sliding member comprises a ruler section, advantageously of elongate shape, comprising a graduated scale. The other one of the base and the sliding member is configured to co-operate with the graduated scale to perform a measurement of a distance of movement of the sliding member and the instrument relative to the base. In some examples, this is accomplished by providing a mark or indicator for co-operation with the graduated scale. In some examples, an edge of the other one of the base and sliding member can be used for this purpose.

One advantage of devices according to the present disclosure is that it is of great simplicity and hence that it can be manufactured at lows cost. Furthermore, due to its simplicity, it can be cleaned easily, which is of great advantage in medical/surgical applications. Yet additionally, the provision of two members, viz. a base and a sliding member, which can move relative to one another, combined with the coupling part allowing releasable engagement with the instrument, allows the device to be used with any available instrument without requiring special provisions for the instruments and guides.

The coupling part is releasable. Advantageously, the coupling part is configured to be released from the instrument by a movement cross to a longitudinal axis of the instrument. In some examples, the coupling part is configured to encircle the instrument only on a portion of its circumference. Therefore, after performing a measurement, the sliding member and possibly the entire device can be set aside or removed from the guide and instrument without causing any hindrance to the further surgical procedure. Additionally, the two-part structure of the device allows even to easily couple the device to the manipulator and the instrument during an intervention for performing unforeseen measurements.

Advantageously, the coupling part comprises or consists of a grasp section fixed to the sliding member. The grasp section advantageously refers to a part of the sliding member that is configured to be operated by an operator's hand, in particular by one or two fingers. The grasp section advantageously comprises a groove adapted to receive the instrument, such that a top portion of an outer surface section of the instrument protrudes from, or is exposed in the groove in a manner accessible by a practitioner's first finger. In some examples, the grasp section comprises a possibly tag-shaped body and the groove is arranged on an outer surface of the tag-shaped body, such as on a top surface. In further advantageous examples, the grasp section comprises a through-hole in a bottom section of the groove, e.g. a through-hole through the tag-shaped body. The through-hole advantageously allows a bottom portion of the outer surface section of the instrument received in the groove to be accessible by a practitioner's second finger. Advantageously, exerting pressure with the first finger and the second finger on respectively the top portion and the bottom portion of the outer surface section of the instrument when received in the groove makes the sliding member move integrally with the instrument. Coupling parts of the above kinds are extremely simple and provide for great ease of use and flexibility. Other examples of suitable coupling parts can comprise a clasp, particularly a spring clasp, a snap fit connector, or other possibly threaded locking connectors, e.g. comprising a locking screw connector for fastening/locking the instrument to the sliding member.

Advantageously, the sliding member comprises an elongate strip or rod on which the graduated scale is provided. Advantageously, the elongate strip is configured to translate along an axis of the translational joint with respect to the base. By so doing, the elongate strip can be slid outwardly from the base when performing a measurement and inwardly again when not needed, resulting in a more compact device which furthermore does not hinder operations when not in use.

Advantageously, the coupling part is configured to engage the instrument at a spaced apart, e.g. elevated, position with respect to the graduated scale. Advantageously, when the coupling part engages the instrument, a longitudinal axis of the coupling part or of the instrument is offset with respect to an axis of translation of the translational joint. In some examples, the coupling part is arranged at a first end of the sliding member. The elongate strip or rod, on which the graduated scale can be provided, is arranged at a second end of the sliding member opposite the first end. A shoulder connects the elongate strip to the coupling part. By engaging the instrument at a spaced apart position with respect to the graduated scale allows to easily keep the scale visible to the eye for performing a measurement. Furthermore, it allows to keep the instrument aligned with the inlet port of the manipulator while attaching the base at any other convenient position. Additionally, it allows the device (sliding member and/or base) to be easily removed from the guide and instrument). In some examples, the attachment means is configured to attach the base to the guide such that an axis of the translational joint is aligned with a longitudinal axis of the inlet port. Alternatively, or in addition, the attachment means is configured to attach the base to the guide such that an axis of the translational joint is aligned with an axis of the coupling part corresponding to a longitudinal axis of the instrument when engaged, such as the axis of the groove of the grasp section.

The translational joint is advantageously a linear motion joint, advantageously a prismatic joint, ensuring linear motion of the sliding member relative to the base, advantageously maintaining alignment of the instrument with the inlet port. The prismatic joint further prevents rotation about the axis of motion (translation). In some examples, either one of the base and the sliding member comprises a body, wherein the body comprises at least one through-hole forming a seat of the translational joint. The through-hole can be configured to receive an elongate section of the other one of the base and sliding member. Particularly the base comprises a body provided with at least one through-hole configured to slidingly receive the elongate strip which can comprise the graduated scale. The through-hole and the elongate section can have mating cross-sections, which can be of a substantially non-circular, such as polygonal, e.g. rectangular shape. Advantageously, an edge of the through-hole can be used as the mark or indicator for taking measurements on the graduated scale.

According to another aspect of the present disclosure, there is provided an assembly comprising the guide, the instrument and the device as substantially described herein.

A method of measuring a longitudinal size of a feature inside a body and/or of a distance of travel or movement of an elongate instrument is described herein. Such methods make use of devices according to the present disclosure.

### Brief Description of Drawings

Aspects of the disclosure will now be described in more detail with reference to the appended drawings, wherein same reference numerals illustrate same features.
Figures 1A and 1B represent an embodiment of a device for measuring the longitudinal size of an anatomical feature inside a patient according to the disclosure.
Figure 2 represents perspective views of the base and the sliding member of measuring device of Figs. 1A-B in isolation.
Figure 3A shows a further embodiment of a measuring device according to the disclosure.
Figure 3B represents perspective views of the base and the sliding member of the measuring device of Fig. 3A in isolation.
Figures 4A, 4B and 4C represent cross sections of different possible coupling means applicable in measuring devices according the disclosure.
Figure 5 represents a perspective view of another measuring device according to the present disclosure.

### Detailed description

The present invention will now be described more fully hereinafter with reference to the accompanying drawings, in which embodiments of the invention are shown. This invention may however be embodied in many different forms and should not be construed as limited to the embodiments set forth herein; rather, these embodiments are provided for purposes of illustration.

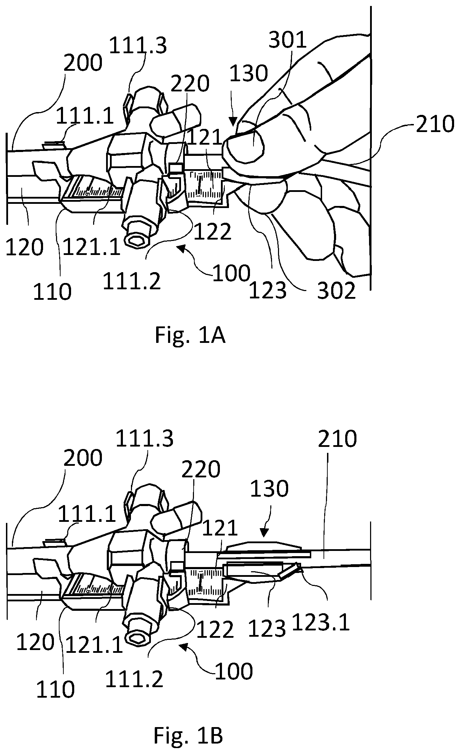

Figures 1A and 1B show a device 100 for measuring the longitudinal size of an anatomical feature inside a patient according to the invention. Said device 100 is also designated as measuring device. Figure 1A differs from Figure 1B in that a hand of a practitioner is present. In Figure 1A, the hand actuates the measuring device 100 during a measurement operation. The measuring device 100 is adapted to be attached to an inlet port 220 of a guide, such as a handle 200 for inserting an instrument 210, in particular a catheter tube, into an internal lumen of the guide which is accessed by the inlet port 220. The guide 200 (handle) can form part of a (medical) manipulator. Such a manipulator is of an elongate shape and comprises an internal lumen, such as a working channel, which is adapted for passing instruments to a target region inside a patient's body. The manipulator can be any suitable endoscopic manipulator, such as an endoscope, or a bronchoscope. The inlet port 220 can be provided at a proximal end of the manipulator, and is typically configured to remain outside the body. The guide can alternatively be any other access point for inserting the instrument inside the body.

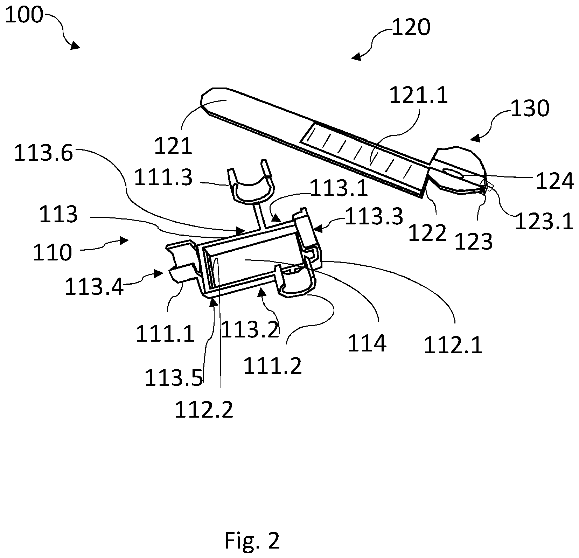

Referring to Fig. 2, the measuring device 100 comprises a base 110 and a sliding member 120. The base 110 is configured to be fixedly and possibly detachably attached to the inlet port 220 of guide 200. The sliding member 120 is configured to translate, e.g. slide, relative to the base along a direction, which corresponds to an axis of the instrument 210 or of the inlet port 220 when base 110 is attached to the guide 200.

The base 110 comprises a body 113 with a top face 113.1, a bottom face 113.2, a proximal face 113.3, a distal face 113.4 opposite the proximal face and two opposite lateral faces 113.5 and 113.6. The body 113 is configured for removable attachment to the medical manipulator 200 via an attachment system comprising one or more attachment means 111.1, 111.2, 111.3. When attached to the guide, the body is positioned such that the distal face 113.4 is facing distally from the operator, towards an elongation of the manipulator, and the proximal face 113.3 is facing towards the operator manipulating the instrument. The proximal and distal faces hence define a longitudinal or axial direction which is substantially aligned with an axis of the instrument or the inlet port (lumen). The lateral faces 113.5, 113.6 are arranged between the distal and proximal faces. Body 113 further comprises a central recess 114 suitably provided on the top face 113.1.

The one or more attachment means can be snap-fit connectors 111.1, 111.2 and 111.3 for advantageously releasable attachment of the base to the inlet port 220, thereby ensuring a firm fixation to the guide 200. The connection between the measuring device 100 and the guide 200 is advantageously resilient so that the measuring device 100 firmly remains in place on the guide 200 without any additional support, thereby ensuring a reduced space occupied by the measuring device 100. Thanks to these measures, the measuring device 100 does not hinder the operations of the practitioner. In Figure 1A and 1B, three snap-fit connectors 111.1, 111.2 and 111.3 are fixed to the handle portion of the manipulator 200 at three separate locations to provide optimal attachment stability. Two of these snap-fit connectors are arranged at the opposite lateral faces 113.5 and 113.6, while a third snap-fit connector is arranged at the distal face 113.4. The three snap-fit connectors are advantageously integral to the base 110 forming a single piece made for instance by plastic molding.

The sliding member 120 can comprise a ruler section 121, an intermediate section 122 and a coupling means 130, in particular a grasp section 123.

The ruler section 121 comprises a graduated scale 121.1, such as printed on a band that is glued on the ruler section 121. As an alternative to a printed band, it can be foreseen to paint and/or engrave the ruler section 121 with graduated marks and signs. The ruler section 121 can be formed as an elongate structure, such as a strip or rod, possibly with a polygonal, such as rectangular cross-section, that is adapted to slide relative to the base 110, although circular cross-sections are possible. To this end, the base 110 comprises a joint configured to constrain motion of the sliding member (ruler section 121) relative to the base to a (linear) translation. By way of example, the joint can be a prismatic joint. In the present example, the prismatic joint comprises a pair of aligned through-holes 112.1 and 112.2 arranged at spaced-apart positions in the body of the base, e.g. at the proximal and distal faces of the base 110. Through-holes 112.1 and 112.2 communicate with the central recess 114 of the body 113. The through-holes have cross-sections mating with the cross-section of the ruler section 121 of the sliding member 120. The ruler section 121 of the sliding member 120 engages in translation in the through-holes 112.1 and 112.2.

The ruler section 121 is arranged at a distal end of the sliding member 120 and engages with the base 110 through the through-holes 112.1, 112.2 of the prismatic joint. The through-holes 112.1 and 112.2 of the prismatic joint communicate with the central recess 114. When the sliding member 120 is inserted through the through-holes of the prismatic joint, a portion of the ruler section will be exposed at the top face 113.1 filling the central recess 114. By so doing, the graduated scale is visible to the operator. An indicator, e.g. shaped as an arrow, can be fixed to the base body to provide a reference mark for the graduated scale. Alternatively, an edge of the central recess 114 or through-hole 112.1 or 112.2 can be used as mark, in particular the edge of through-hole 112.1 at the proximal face 113.3 can be used as reference mark for the graduated scale. It is alternatively possible to arrange the graduated scale on the back face of the ruler section 121 and measurements can be easily read from that side as well, since the back side may be facing the operator.

The grasp section 123 is arranged at an opposite end of the sliding member 120 relative to the ruler section and comprises a petal or tag shaped component that includes a groove 123.1 adapted to receive the instrument 210. The grasp section 123 acts as a coupling means 130 such that, when a practitioner squeezes the instrument 210 and the grasp section 123 between two fingers 301 and 302 as shown in Fig. 1A, the sliding member 120 will move integrally with the instrument 210. When the coupling means 130 is engaged, the instrument 210 and the grasp section 123 are engaged with one another via friction resulting from the compression exerted by the practitioner's fingers 301 and 302. When the instrument 210 is not pressed in the groove 123.1 by the practitioner, the instrument 210 can freely slide relative to the sliding member 120 as shown in Figure 1B, implying that the coupling means 130 is disengaged. The groove 123.1 is aligned with a longitudinal axis of the ruler section 121 and aligned with the prismatic joint 112.1, 112.2 of the base, which also allows to align the instrument 210 with the orifice of the inlet port 220 of the endoscope 200, thereby improving its handling.

Referring to Fig. 2, a through-hole 124 is advantageously arranged in a bottom of groove 123.1. Through-hole 124 exposes a bottom portion of the outer surface portion of the instrument 210 and the outer surface portion of instrument 210 accessible to finger 302. When the instrument 210 and the grasp section 123 are squeezed between fingers 301 and 302, a better clamping is obtained.

The intermediate section 122 connects the ruler section 121 to the grasp section 123. It advantageously comprises a shoulder or flange portion allowing to maintain the instrument 210 at an elevated position relative to the ruler section 121, thereby bridging a possible height difference between the position of the prismatic joint and the entrance of the lumen of the manipulator 200.

It will be convenient to note that the guiding means/prismatic joint and the ruler section can be interchanged between the base and the sliding member, e.g. the ruler section can be provided fixedly attached to the base and the prismatic joint provided on the sliding member. In this case, the sliding member will be configured to slide along the ruler section, and a distance can be determined by means of an indicator or mark provided fixed on the sliding member. One disadvantage of the latter configuration is that the measuring device becomes more bulky since the ruler section will always project out of the base.

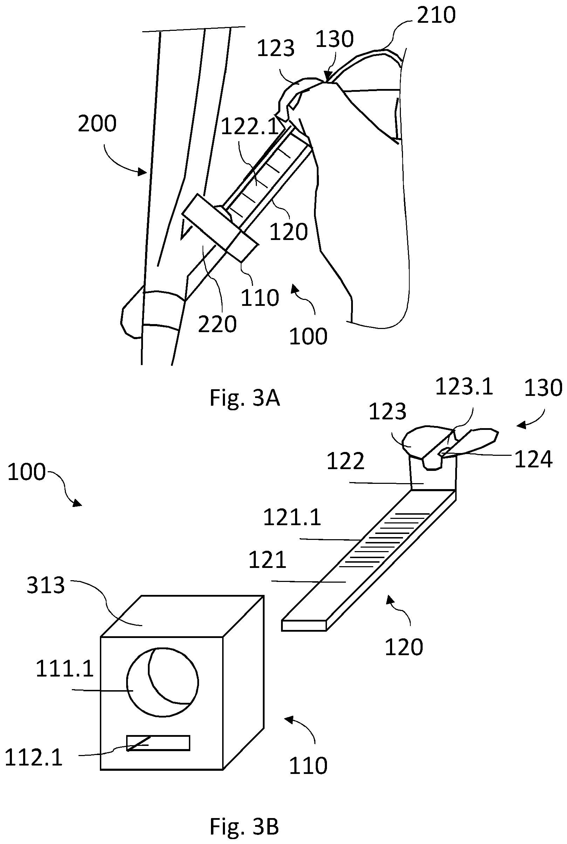

Figures 3A-B show a further embodiment of a measuring device according to the invention. The embodiment of Figs. 3A-B differs from the embodiment of Figs. 1A-B in an alternative structure for the attachment means. In this case, the base 110 comprises a body 313 having prismatic or cylindrical shape, in particular as a right prism with e.g. a rectangular base. The attachment means is provided as a through-bore 111.1 in the body of base 110. The through-bore is sized to snugly fit on an outer circumference of the inlet port 220.

While the through-bore is shown with a circular cross section in Fig. 3B, it is possible to utilize non-circular geometries, which may provide for rotation-locking properties.

A prismatic joint for guiding translational motion of the sliding member 120 with respect to the base 110 is provided as a second through-bore 112.1 through the body 313. The second through-bore 112.1 is configured to slidingly receive the ruler section 121 of the sliding member 120.

Other types of attachment means can be foreseen for attaching the base 110 to the guide, such as a magnetic or strap fixation alone or in combination with above-mentioned attachment means. The selection of a particular type attachment means is not particularly critical and may depend on the design of the medical manipulator 200.

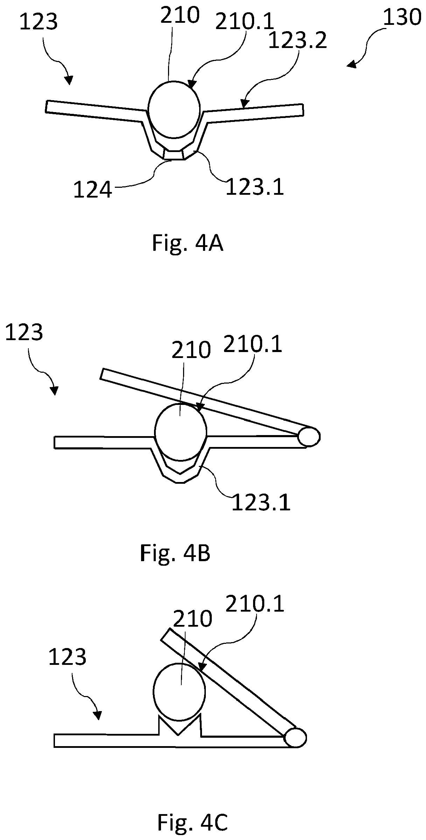

Figure 4A shows a cross section of the grasp section 123 as described above. The coupling means 130, in particular the grasp section 123 comprises a petal or tag shaped component adapted to be easily squeezed between a thumb 301 on the concave side and the index finger 302 on the convex side. The groove 123.1 is sized such that a top portion 210.1 of a section of the outer surface of instrument 210 protrudes from a top surface 123.2 of grasp section 123. It is alternatively possible to have the top portion 210.1 be flush with the surface 123.2 or even completely received in the groove 123.1, as long as the top portion 210.1 remains accessible to the finger 301. The petal or tag-shaped component can have any suitable shape to ease manufacturing. Generally, the groove 123.1 provides a loose fit for instrument 210. It will be convenient to note that the coupling means 130 is not limited to these designs. Other configurations, for instance shown in Figures 4B and 4C, comprise a claw or clasp, which may or may not be spring-biased, to enhance the friction between the instrument 210 and the sliding member 120, in particular by providing additional engagement on the top portion 210.1 of the instrument 210. The embodiments shown in Figure 4B and 4C can be advantageous for hygienic requirements because the instrument 210 is not in direct contact with the practitioner's hand. Alternative embodiments include screw-type locking connectors, in which a screw tightly fastens the instrument against a surface of the grasp section, and snap-fit connectors, e.g. similar to connectors 111.1, 111.2, 111.3.

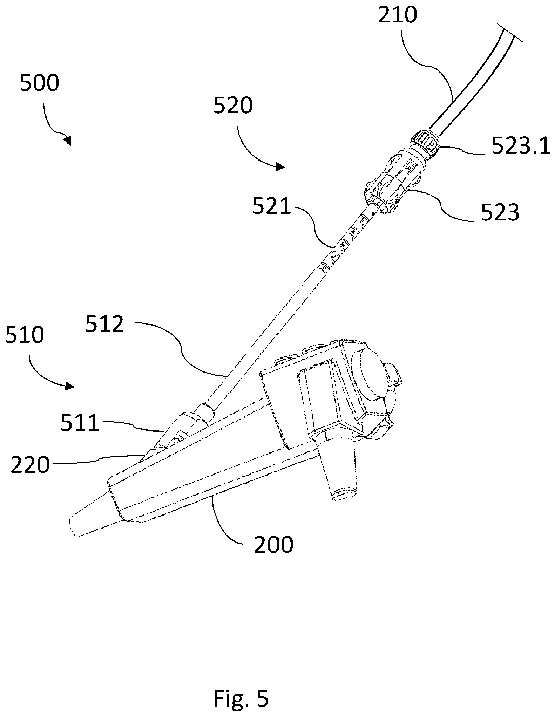

Referring to Fig. 5, another example of a measuring device 500 according to aspects of the present disclosure differs from the examples described previously in that an axis of the translational joint between the base 510 and the sliding member 520 is concentric with the longitudinal axis of the instrument 210. Base 510 comprises a first tubular member 512 and sliding member 520 comprises a second tubular member 521. One of both tubular members is arranged to slide over the other one and both can receive the instrument inside. In the example of Fig. 5, the first tubular member 512 has a larger inner diameter than the outer diameter of the second tubular member 521. First tubular member 512 hence receives second tubular member 521 and both form a translational joint. A graduated scale can be provided on the outer surface of the second tubular member 521 to be visible when the tubular member 521 is retracted. In an alternative configuration (not shown), the second tubular member has a larger inner diameter than the outer diameter of the first tubular member and slides over it. The graduated scale can be conveniently provided on the first tubular member.

A locking member 511, such as a threaded lock or a snap-fit lock is provided at a distal end of the first tubular member 512 for attaching the base 510 to an inlet port 220 of the guide 200, which is shown here as a handle of an endoscopic manipulator. A grasp section 523 provided at a proximal end of the second tubular member 521 allows easy engagement of sliding member 520 by an operator's hand. The grasp section 523 is provided with a coupling member 523.1 for detachable or releasable attachment of the instrument 210 to the sliding member 520. Coupling member 523.1 can be any suitable connector for frictionally engaging with the instrument as described above, so as to let sliding member 520 move integrally with the instrument 210.

Measuring the translation distance of the instrument through the manipulator, e.g. for determining the length of a stenosis, can be performed as follows. If the stenosis is small enough to allow passage of the instrument (catheter tube) tip through it, the catheter-tube tip may be passed across the stenotic lesion. By estimating with the catheter tip the location of the distal end of the lesion, and thereafter withdrawing the catheter-tube tip to the estimated location of the proximal end of the stenosis, the length of the stenosis can be estimated. The catheter-tube tip is positioned at the distal end of the lesion and the sliding member 120 is positioned in an advanced position (e.g. with shoulder 122 abutting against base body 113), with a reference of the graduated scale corresponding to the mark or indicator of the base. The practitioner can then hold the grasp section 123 and the catheter tube 210 together by squeezing between two fingers. While doing so, the practitioner slowly retracts the catheter tube 210 together (integrally) with the sliding member up to the point where the catheter tip exits the stenosis region. A length measurement can be derived by reading the markings on the ruler section 121 (graduated scale). The marking of the ruler section 121 (see Figure 1 A) coinciding with the abutment face of the base 110 directly indicates the length of the stenosis once the proximal end of the stenosis is reached.

In another example, the length of the stenosis can be measured while displacing the catheter tip from the proximal end to the distal end of the lesion. In this case, the sliding member 120 is positioned in a retracted position, e.g. with shoulder 122 arranged remote from base body 113. Equally, the scale 121.1 of ruler section 121 can be inverted in comparison to the previous case so that the marking of the ruler section 121 coinciding with the abutment face (proximal face 113.3) of the base 110 indicates the real displacement of the sliding member 123 and therefore the catheter-tube tip. Alternatively, the ruler section 121 may comprise two opposed scales so that the practitioner has more flexibility and reliability and the measurement can be performed in both directions.

While the invention has been illustrated and described in detail in the drawings and foregoing description, such illustration and description are to be considered illustrative or exemplary and not restrictive. Other variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. The mere fact that certain measures are recited in different mutually dependent claims does not indicate that a combination of these measures cannot be used to advantage. Any reference signs in the claims should not be construed as limiting the scope.

## Claims

1. A device (100, 500) for measuring advancement of an instrument (210) inside a body, the device being configured to be detachably attached to a guide (200) for the instrument, the guide comprising an inlet port (220) through which the instrument (210) can access the body, the device comprising:
a base (110, 510) comprising attachment means (111.1, 111.2, 111.3, 511) for attachment to the guide (200) so that the base (110, 510) remains fixed with respect to the inlet port (220) when attached,
**characterised in that** the device comprises:
a sliding member (120, 520) comprising a coupling means (130, 523.1) configured to releasably engage with the instrument (210) so that the sliding member moves integrally with the instrument when coupled,
wherein the device comprises a translational joint (112.1, 112.2) allowing the sliding member to translate relative to the base,
wherein one of the base and the sliding member comprise a ruler section (121) with a graduated scale configured to co-operate with the other one of the base and the sliding member to perform a measurement of a distance of movement.

2. Device according to Claim 1, wherein the coupling means (130) comprises a grasp section (123) fixed to the sliding member (120), the grasp section comprising a groove (123.1) adapted to receive the instrument (210), such that a top portion of an outer surface section of the instrument (210) protrudes from, or is exposed in the groove (123.1), in a manner accessible by a practitioner's first finger (301).

3. Device according to Claim 2, wherein the grasp section comprises a through-hole in a bottom section of the groove (123.1), such that a bottom portion of the outer surface section of the instrument received in the groove is accessible by a practitioner's second finger (302).

4. Device according to Claim 3, wherein exerting pressure with the first finger and the second finger on respectively the top portion and the bottom portion of the outer surface section of the instrument (210) when received in the groove makes the instrument and the sliding member move integrally.

5. Device according to any one of the claims 2 to 4, wherein a longitudinal axis of the groove (123.1) is parallel to an axis of translation of the translational joint.

6. Device according to claim 1, wherein the coupling means comprises a locking member (523.1).

7. Device according to any one of the preceding claims, wherein the sliding member comprises an elongate strip (121) on which the graduated scale is provided, the elongate strip being configured to translate along an axis of the translational joint with respect to the base.

8. Device according to any one of the preceding claims, wherein an axis of translation of the sliding member (120) is offset with respect to a longitudinal axis of the inlet port.

9. Device according to any one of the preceding claims, wherein the coupling means (130) is arranged at a first end of the sliding member (120), wherein the sliding member comprises an elongate strip (121) on which the graduated scale is provided, wherein the elongate strip (121) is arranged at a second end of the sliding member opposite the first end and wherein the sliding member comprises a shoulder (122) connecting the elongate strip (121) and the coupling means (130), the shoulder projecting from the elongate strip in a direction cross to an axis of translation of the translational joint.

10. Device according to any one of the previous claims, wherein the translational joint is a prismatic joint.

11. Device according to any one of the preceding claims, wherein one of the base and the sliding member comprises a body (113, 313, 512), wherein the body comprises at least one through-hole (112.1, 112.2) forming a seat of the prismatic joint, wherein the through-hole is configured to receive an elongate section (121, 521) of the other one of the base and the sliding member.

12. Device according to claim 11, wherein the elongate section of the sliding member has a polygonal cross-section.

13. Device according to any one of the previous claims, wherein the attachment means (111.1, 511) is configured to attach the base to the guide (200) such that an axis of translation of the translational joint is parallel with a longitudinal axis of the inlet port.

14. Device according to any one of the previous claims, wherein the attachment means (111.1, 111.2, 111.3) is configured for detachable attachment, preferably the attachment means comprises one or a combination of: a snap fit connector and a threaded locking connector.

15. Assembly, comprising an instrument (210), a guide (200) for the instrument and a device (100, 500) according to any one of the previous claims, wherein the guide (200), in particular an endoscope, comprises an inlet port (220) for receiving the instrument (210), in particular a catheter, therethrough, preferably wherein the guide (200) further comprises an internal lumen communicating with the inlet port for guiding the instrument.
